(19) Europäisches Patentamt
European Patent Office
Office européen des brevets

(11) Publication number: 0 276 807 A2

(12) EUROPEAN PATENT APPLICATION

(21) Application number: 88101071.4

(22) Date of filing: 26.01.88

(51) Int. Cl.4: A61K 31/365, A61K 31/35, A61K 31/215, A61K 31/19, //(A61K31/365,31:35,31:215, 31:19)

(30) Priority: 27.01.87 US 7061

(43) Date of publication of application: 03.08.88 Bulletin 88/31

(84) Designated Contracting States: ES GR

(71) Applicant: WARNER-LAMBERT COMPANY
201 Tabor Road
Morris Plains New Jersey 07950(US)

(72) Inventor: Fawzi, Mahdi B.
11 Timberline Drive
Flanders New Jersey 07836(US)
Inventor: Newton, Roger S.
1425 Bardstown Trail
Ann Arbor, Michigan 48105(US)
Inventor: Roth, Bruce D.
1440 King George Blvd.
Ann Arbor, Michigan 48104(US)
Inventor: Yakatan, Gerald J.
13813 Boquita Drive
Del Mar California 92014(US)

(74) Representative: Mansmann, Ivo
c/o Gödecke AG - Patentabteilung Postfach
569 - Mooswaldallee 1-9
D-7800 Freiburg(DE)

(54) **Lipid regulating compositions.**

(57) Single dose formulations containing 5-(2,5-dimethylphenoxy)-2,2-dimethylpentanoic acid and an HMG-CoA reductase inhibitor are effective pharmacological formulations for regulating blood serum lipid and cholesterol levels.

EP 0 276 807 A2

# LIPID REGULATING COMPOSITIONS

## Background of the Invention

The present invention is related to pharmaceutical compositions and methods of treatment. More particularly it concerns pharmaceutical compositions comprising combinations of blood serum lipid and cholesterol regulating agents.

In recent years the role which elevated blood plasma levels of cholesterol plays in pathological conditions in man has received much attention. Deposits of cholesterol in the vascular system have been indicated as causative of a variety of pathological conditions including coronary heart disease.

Initially, studies of this problem were directed toward finding therapeutic agents which are effective in lowering total serum cholesterol levels. However, it has since been discovered that the mechanism by which cholesterol is transported in the blood and deposited as plaques on vascular walls is quite complex. Current understanding of the problem differentiates between the different forms of lipoprotein cholesterol which circulate in the blood stream and it is now believed that effective approaches to the control of blood serum cholesterol involve more than merely reducing the levels of total serum cholesterol.

For example, it is now known that cholesterol is transported in the blood stream in the form of complex particles consisting of a core of cholesteryl esters plus triglycerides and an exterior consisting primarily of phospholipids and a variety of types of protein which are recognized by specific receptors. For example, cholesterol is carried to the sites of deposit in blood vessels in the form of low density lipoprotein cholesterol (LDL cholesterol) and away from such sites of deposit by high density lipoprotein cholesterol (HDL cholesterol).

Following these discoveries, the search for therapeutic agents which control serum cholesterol has increasingly turned to finding compounds which are more selective in their action; that is, agents which are effective both in elevating the blood serum levels of HDL cholesterol and/or lowering the levels of LDL cholesterol, and in lower serum triglycerides.

One approach to lowering total and LDL-cholesterol levels in blood serum has centered around the discovery of therapeutic agents which inhibit the enzyme 3-hydroxy-3-methylglutaryl coenzyme A reductase (HMG-CoA reductase). This enzyme acts in the metabolic pathway involving the biosynthesis of cholesterol, and it is known that inhibitors of HMG-CoA reductase are effective in lowering the level of blood plasma cholesterol, especially LDL-cholesterol, in man (cf. M. S. Brown and J. L. Goldstein, New England Journal of Medicine, 305 (9): 515-517 (1981).

In an alternative approach to the control of serum lipoproteins and cholesterol, it has been found that 5-(2,5-dimethylphenoxy)-2,2-dimethylpentanoic acid (commonly known as gemfibrozil) acts to elevate plasma levels of HDL-cholesterol and to decrease plasma triglycerides. This pharmacological action is an effective method of treating a variety of primary and secondary dislipoproteinemias. (See Saku et al., J. Clin. Invest., 75: 1702-1712 (1985)).

Some attempts have been made to find effective combinations of therapeutic agents to control serum cholesterol and lipid levels. United States Patent 3,846,541 discloses the coadministration of chlorophenoxy isobutyric acid and its esters with a bile acid sequestering agent. Such combinations result in some lowering of low-density and total cholesterol, but result in compensatory increases in cholesterol biosynthesis.

European Patent Application 0 010 299 discloses combinations of HMG-CoA reductase inhibitors and bile acid sequestrants which are effective in lowering LDL-cholesterol but have minimal effect on HDL-cholesterol.

Because HDL-cholesterol is involved in transporting cholesterol away from the sites of vascular deposit and LDL-cholesterol is involvled in transporting cholesterol to such sites of deposit, there is a need for effective hypolipidemic agents which act by simultaneously elevating HDL-cholesterol, while lowering LDL-cholesterol and serum triglyceride levels.

## Summary of the Invention

It has been found in accordance with the present invention that pharmaceutical compositions combining 5-(2,5-dimethylphenoxy)-2,2-dimethylpentanoic acid (gemfibrozil), its lower alkyl esters and pharmaceutically acceptable acid addition salts together with one or more HMG-CoA reductase inhibitors are effective

hypolipidemic agents which elevate HDL-cholesterol while concommittantly lowering levels of LDL-cholesterol and serum triglycerides.

The pharmaceutical compositions of the present invention comprise a first component comprising 5-(2,5-dimethylphenoxy)-2,2-dimethylpentanoic acid (more commonly known as gemfibrozil) or a lower alkyl ester or pharmaceutically acceptable acid addition salt thereof together with a second component comprising an HMG-CoA reductase inhibitor selected from

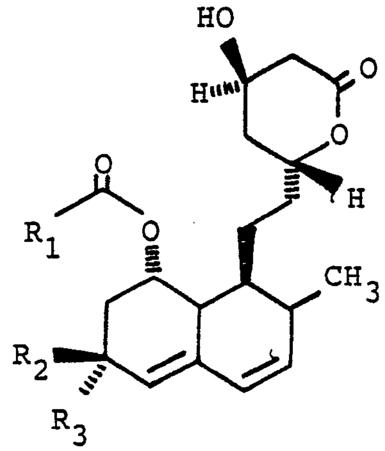

I

or

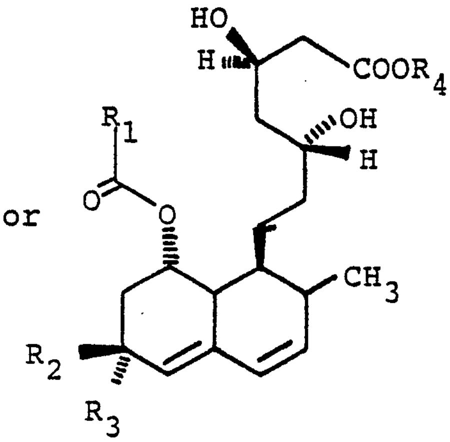

II

where $R_1$ is selected from $-\overset{CH_3}{\overset{|}{C}H}-CH_2CH_3$, and

$-\overset{CH_3}{\overset{|}{\underset{CH_3}{\underset{|}{C}}H}}-CH_2CH_3$. $R_2$ is hydrogen or hydroxy, $R_3$ is hydrogen or methyl, and $R_4$ is lower alkyl or hydrogen or a pharmaceutically acceptable base addition salt thereof. The first and second components of the pharmaceutical composition are present in a weight ratio of respectively between 5:1 and 200:1.

## Detailed Description

Particularly preferred HMG-CoA reductase inhibitors for incorporation into the pharmaceutical compositions of this invention together with gemfibrozil are the compounds of formula I above where $R_1$ is (S)-1-methylpropyl or 1,1-dimethylpropyl, $R_2$ is hydrogen and $R_3$ is methyl, the compound where $R_1$ is (S)-1-methylpropyl, $R_2$ is hydroxy and $R_3$ is hydrogen, and the compound where $R_1$ is (S)-1-methylpropyl, and both $R_2$ and $R_3$ are hydrogen. Also preferred are the corresponding lactone ring-opened dihydroxy acids of formula II where the values of $R_1$, $R_2$ and $R_3$ are as just previously defined.

As used throughout this specification and the appended claims, the term "lower alkyl" means alkyl of from one to four carbon atoms and includes methyl, ethyl, propyl, isopropyl, n-butyl, sec-butyl, iso-butyl, and tert-butyl.

The compound 5-(2,5-dimethylphenoxy)-2,2-dimethylpentanoic acid (gemfibrozil) and its method of preparation are disclosed in United States Patent 3,674,836.

The compound of formula I above where $R_1$ is (S)-1-methylpropyl, $R_2$ is hydrogen and $R_3$ is methyl has the systematic name [1S-[1α(R*),3α,7β,8β(2S*,4S*),-8aβ]]-1,2,3,7,8,8a-hexahydro-3,7-dimethyl-8-[2-(tetrahydro-4-hydroxy-6-oxo-2H-pyran-2-yl)ethyl]-1-naphthalenyl 2-methylbutanoate, and is more commonly known by its trivial name, mevinolin. Mevinolin is a potent inhibitor of HMG-CoA, having an $IC_{50}$ value of about 2.2 nanomolar. The compound is the product of fermentation and its preparation and isolation are described in United States Patents 4,231,938; 4,294,926; and 4,342,767.

The lactone ring-opened dihydroxy acid corresponding to mevinolin has the systematic name [1S-[1α-

(βS*,δS*),2α,6β,8β(R*)8aα]]-1,2,6,7,8,8a-hexahydro-β,δ-dihydroxy-2,6-dimethyl-8-(2-methyl-1-oxobutoxy)-1-naphthaleneheptanoic acid, and is more commonly known by its trivial name mevinolinic acid. Mevinolinic acid possesses an $IC_{50}$ value of about 5.6 nanomolar for inhibition of HMG-CoA reductase. The production of mevinolinic acid and its salts is described in United States Patents 4,319,039 and 4,342,767.

The compound of formula I above where $R_1$ is 1,1-dimethylpropyl, $R_2$ is hydrogen and $R_3$ is methyl has the systematic name [1S-[1α,3α,7β,8β(2S*,4S*)8aβ]]-1,2,3,7,8,8a-hexahydro-3,7-dimethyl-8-[2-(tetrahydro-4-hydroxy-6-oxo-2H-pyran-2-yl)ethyl]-1-naphthalenyl 2,2-dimethylbutanoate, and is more commonly known by its trivial name, synvinolin. The lactone ring-opened dihydroxy acid corresponding to synvinolin has the systematic name [1S-[1α(βS*,δS*),2α,6β,8β,8aα]]-8-(2,2-dimethyl-1-oxobutoxy)-1,2,6,7,8,8a-hexahydro-β,δ-dihydroxy-2,6-dimethyl-1-naphthaleneheptanoic acid. The preparation of synvinolin and the corresponding lactone ring-opened dihydroxy acid are described in United States Patent 4,444,784.

The compound of formula I above where $R_1$ is (S)-1-methylpropyl, $R_2$ is hydroxy and $R_3$ is hydrogen has the systematic name [1S-[1α(R*),3β,7β,8β(2S*,4S*),-8aβ]]-1,2,3,7,8,8a-hexahydro-3-hydroxy-7-methyl-8-[2-(tetrahydro-4-hydroxy-6-oxo-2H-pyran-2-yl)ethyl]-1-naphthalenyl 2-methylbutanoate. The corresponding lactone-ring-opened trihydroxy acid has the systematic name [1S-[1α(βS*,δS*),2α,6α,8β(R*),8aα]]-1,2,6,7,8,8a-hexahydro-β,δ,6-trihydroxy-2-methyl-8-(2-methyl-1-oxobutoxy)-1-naphthaleneheptanoic acid. The production of both compounds is described in United States Patent 4,346,227.

The compound of formula I above where $R_1$ is (S)-1-methylpropyl, and $R_2$ and $R_3$ are both hydrogen has the systematic name [1S-[1α(R*),7β,8β(2S*,4S*),-8aβ]]-1,2,3,7,8,8a-hexahydro-7-methyl-8-[2-(tetrahydro-4-hydroxy-6-oxo-2H-pyran-2-yl)ethyl]-1-naphthanlenyl 2-methylbutanoate, and is more commonly known by its trivial name, compactin. The production of compactin is described in United States patents 3,983,140 and 4,049,495. The lactone ring-opened dihydroxy acid corresponding to compactin has the systematic name [1S-[1α(βS*,δS*),2α,8β(R*),-8aα]]-1,2,6,7,8,8a-hexahydro-β,δ-dihydroxy-2-methyl-8-(2-methyl-1-oxobutoxy)-1-naphthaleneheptanoic acid.

Gemfibrozil and the ring-opened hydroxy acids of structural formula II above may be used in their free acid form or in the form of a lower alkyl ester or a pharmaceutically acceptable metal or amine salt in the pharmaceutical compositions of the present invention.

Esters are prepared by reacting the free acids with the desired lower alkyl alcohol in the presence of an ester condensation agent such as carbonyldiimidazole, dicyclohexylcarbodiimide and the like. Alternatively, the lactone compounds of formula I may be simultaneously ring-opened and esterified by the action of a sodium salt of the desired lower alkyl alcohol in the alcohol, serving as solvent.

These acids also react to form pharmaceutically acceptable metal and amine salts. The term "pharmaceutically acceptable metal salt" contemplates salts formed with the sodium, potassium, calcium, magnesium, aluminum, iron, and zinc ions. The term "pharmaceutically acceptable amine salt" contemplates salts with ammonia and organic nitrogenous bases strong enough to form salts with carboxylic acids. Bases useful for the formation of pharmaceutically acceptable nontoxic base addition salts of compounds of the present invention form a class whose limits are readily understood by those skilled in the art. See, for example, S. M. Berge et al., J. Pharm. Sci., 66: 1-19 (1977).

The free acid form of compounds of the present invention may be regenerated from the salt form, if desired, by contacting the salt with a dilute aqueous solution of an acid such as hydrochloric acid.

The base addition salts may differ from the free acid forms of the compounds of this invention in such physical characteristics as solubility and melting point, but are otherwise considered equivalent to the free acid form for the purposes of this invention.

The compounds of the present invention may exist in solvated or unsolvated form. In general, the solvated forms with pharmaceutically acceptable solvents such as water, ethanol and the like, are equivalent to the unsolvated forms for the purposes of this invention.

For preparing pharmaceutical compositions from the compounds of this invention, inert, pharmaceutically acceptable carriers can be either solid or liquid. Solid form preparations include powders, tablets, dispersable granules, capsules, and sachets.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active compound is mixed with the carrier having the necessary binding properties in suitable proportions and compacted in the shape and size desired.

Powders and tablets preferably contain between about 5 to about 95% by weight of the active ingredient. Suitable carriers are magnesium carbonate, calcium stearate, magnesium stearate, talc, lactose, sugar, pectin, dextrin, starch, tragacanth, methyl cellulose, sodium carboxymethyl cellulose, and the like.

The term "preparation" is intended to include the formulation of the active compound with encapsulating material as a carrier providing a capsule in which the active component (with or without other carriers) is surrounded by a carrier, which is thus in association with it. In a similar manner, sachets are also included. Tablets, powders, sachets, and capsules can be used as solid dosage forms suitable for oral administration.

Liquid form preparations include solutions, suspensions and emulsions suitable for oral administration. Aqueous solutions for oral administration can be prepared by dissolving the active compound in water and adding suitable flavorants, coloring agents, stabilizers, and thickening agents as desired. Ethanol, propylene glycol and other pharmaceutically acceptable non-aqueous solvents may be added to improve solubility of the active components. Aqueous suspensions for oral use can be made by dispersing the finely divided active component in water together with a viscous material such as natural or synthetic gums, resins, methyl cellulose, sodium carboxymethyl cellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, sachet, or tablet itself, or it can be the appropriate number of any of these packaged forms.

In therapeutic use as hypolipidemic or hypocholesterolemic agents, the pharmaceutical compositions of this invention are administered to the patient at dosage levels of from 300 to 1200 mg per day of 5-(2,5-dimethylphenoxy)-2,2-dimethylpentanoic acid (gemfibrozil) and from 5 to 80 mg per day of HMG-CoA reductase inhibitor in a combined formulation. For dosage forms which include up to 900 mg of gemfibrozil, the dosage may be in a single daily dose, preferably in a sustained-release formulation. For dosage forms containing up to 600 mg of gemfibrozil, the dosage may be twice daily. The preferred dose is 600 mg of gemfibrozil and 40 mg of HMG-CoA reductase inhibitor, administered once daily.

The dosages, however, may be varied depending upon the requirements of the patient, the severity of the condition being treated, and the compound being employed. Determination of optimum dosages for a particular situation is within the skill of the physician.

The following examples illustrate particular methods for preparing pharmaceutical compositions in accordance with this invention. These examples are illustrative and are not to be read as necessarily limiting the scope of the invention as it is defined by the appended claims.

Example 1

Capsule Formulation

To prepare 1000 No. 0 gelatine capsules, each containing 300 mg of gemfibrozil and 20 mg of HMG-CoA reductase inhibitor, the following ingredients are employed.

Ingredients

1. Gemfibrozil, 300 g.
2. HMG-CoA reductase inhibitor, 20 g.
3. Finely divided silicon dioxide, 15 g.
4. Corn starch N.F., 141 g.
5. Polysorbate 80 N.F., 4 g.
6. Purified water, 80 ml.

Method of Formulation

Step A: Blend, after milling, ingredients 1, 2, 3, and 4 in a suitable mixer.

Step B: Dissolve 5 in 6.

Step C: Granulate the blended ingredients of Step A together with the ingredients of Step B in a suitable blender.

Step D: Dry the granulation at 40°C overnight.

Step E: Mill the dried granulation of Step D using a Fitzmill No. 2RH screen with impact forward at high speed.

Step F: Fill No.0 capsules with 480 mg each of the milled mixture from Step E.

Example 2

Immediate-release Tablet Formulation

To prepare 1000 immediate-release tablets each containing 300 mg each of gemfibrozil and 10 mg of HMG-CoA reductase inhibitor, the following ingredients are employed.

Ingredients

1. Gemfibrozil, 300 g.
2. HMG-CoA reductase inhibitor, 10 g.
3. Finely divided silicon dioxide, 6 g.
4. Pregelatinized starch 1551 N.F., 71 g.
5. Hydroxypropylcellulose, 8 g.
6. Polysorbate 80 N.F., 3 g.
7. Purified water, 75 ml.
8. Cellulose microcrystalline N.F., 33 g.
9. Finely divided silicon dioxide, 5 g.
10. Calcium stearate, 5 g.

Method of Formulation

Step A: Mill ingredients 1, 2, 3 and 5 through a No. 0 Screen.

Step B: B. Dissolve 6 in 7 and mix well.

Step C: Blend ingredients 1, 2, 3, 4, and 5, in a high intensity type mixer, using mixer and granulator modes for 3 minutes.

Step D: Granulate the mixture of ingredients from Step C together with the mixture from Step D. Add additional water if needed.

Step E: Spread the moist granulation from Step D on paper-lined trays and dry at 40°C in a forced-air oven to an LOD of 2-3%.

Step F: Pass dried granulation from Step E, together with ingredients 9 and 10 through a Fitzmill No. 2RH screen, knives forward, medium speed.

Step G: Add ingredient 8 to the milled granulation from Step F and blend well.

Step H: Compress tablets on appropriate punches to a 10-12 Kp hardness and about 0.33 inch guage.

Example 3

Immediate-release Tablet Formulation

To prepare 1000 immediate-release tablets each containing 450 mg of gemfibrozil and 5 mg of HMG-CoA reductase inhibitor, the procedure of Example 2 is followed utilizing the following ingredients.

Ingredients

1. Gemfibrozil, 450 g.
2. HMG-CoA reductase inhibitor, 5 g.
3. Finely divided silicon dioxide, 9 g.
4. Pregelatinized starch 1551 N.F., 106 g.

5. Hydroxypropylcellulose, 12 g.
6. Polysorbate 80 N.F., 5 g.
7. Purified water, 100 ml.
8. Cellulose microcrystalline N.F., 49 g.
9. Finely divided silicon dioxide, 8 g.
10. Calcium stearate, 8 g.

Example 4

Immediate-release Tablet Formulation

To prepare 1000 immediate-release tablets each containing 450 mg of gemfibrozil and 40 mg of HMG-CoA reductase inhibitor, the procedure of Example 3 is followed utilizing the following ingredients.

Ingredients

1. Gemfibrozil, 450 g.
2. HMG-CoA reductase inhibitor, 40 g.
3. Finely divided silicon dioxide, 9 g.
4. Pregelatinized starch 1551 N.F., 106 g.
5. Hydroxypropylcellulose, 12 g.
6. Polysorbate 80 N.F., 5 g.
7. Purified water, 100 ml.
8. Cellulose microcrystalline N.F., 49 g.
9. Finely divided silicon dioxide, 8 g.
10. Calcium stearate, 8 g.

Example 5

Immediate-release Tablet Formulation

To prepare 1000 immediate-release tablets each containing 600 mg of gemfibrozil and 50 mg of HMG-CoA reductase inhibitor, the procedure of Example 3 is followed utilizing the following ingredients.

Ingredients

1. Gemfibrozil, 600 g.
2. HMG-CoA reductase inhibitor, 50 g.
3. Finely divided silicon dioxide, 12 g.
4. Pregelatinized starch 1551 N.F., 141 g.
5. Hydroxypropylcellulose, 16 g.
6. Polysorbate 80 N.F., 6 g.
7. Purified water, 150 ml.
8. Cellulose microcrystalline N.F., 65 g.
9. Finely divided silicon dioxide, 10 g.
10. Calcium stearate, 10 g.

Example 6

Immediate-release Tablet Formulation

To prepare 1000 immediate-release tablets each containing 600 mg of gemfibrozil and 5 mg of HMG-CoA reductase inhibitor, the procedure of Example 3 is followed utilizing the following ingredients.

Ingredients

1. Gemfibrozil, 600 g.
2. HMG-CoA reductase inhibitor, 5 g.
3. Finely divided silicon dioxide, 12 g.
4. Pregelatinized starch 1551 N.F., 141 g.
5. Hydroxypropylcellulose, 16 g.
6. Polysorbate 80 N.F., 6 g.
7. Purified water, 150 ml.
8. Cellulose microcrystalline N.F., 65 g.
9. Finely divided silicon dioxide, 10 g.
10. Calcium stearate, 10 g.

Example 7

Immediate-release Tablet Formulation

To prepare 1000 immediate-release tablets each containing 600 mg of gemfibrozil and 20 mg of HMG-CoA reductase inhibitor, the procedure of Example 3 is followed utilizing the following ingredients.

Ingredients

1. Gemfibrozil, 600 g.
2. HMG-CoA reductase inhibitor, 20 g.
3. Finely divided silicon dioxide, 12 g.
4. Pregelatinized starch 1551 N.F., 141 g.
5. Hydroxypropylcellulose, 16 g.
6. Polysorbate 80 N.F., 7 g.
7. Purified water, 150 ml.
8. Cellulose microcrystalline N.F., 65 g.
9. Finely divided silicon dioxide, 10 g.
10. Calcium stearate, 10 g.

Example 8

Sustained-release Tablet Formulation

To prepare 1000 sustained-release tablets each containing 450 mg of gemfibrozil and 20 mg of HMG-CoA reductase inhibitor, the following ingredients are utilized.

Ingredients

1. Gemfibrozil, 450 g.
2. HMG-CoA reductase inhibitor, 20 g.
3. Microcrystalline cellulose type CL611, 35 g.
4. Cross carmellose sodium NF type A, 5 g.
5. Eudragit E30D, 180 g.
6. Purified water, 35 ml.
7. Talc USP, 5 g.
8. Cross carmellose Na NF type A, 7 g.
9. Ca stearate NF, 5 g.

Method of Formulation

Step A: Load ingredients 1, 2, 3 and 4 into a Collette Gral and mix for 3 minutes with the mixer at 200 rpm and the granulator speed set at 2. Add ingredient 5 while mixing and continue to mix for an additional 2 minute using the same mixing conditions. Use sufficient quantity of purified water to make the granulation.

Step B: Spread the moist granulation on paper lined trays and dry in forced-air oven at 38-40°C to an LOD of less than 1%.

Step C: Add ingredients 7 and 8. Pass the resulting mixture through a Fitzmill No. 2RH screen, knives forward at medium speed.

Step D: Load the granulation into an appropriate blender and tumble blend for five minues.

Step E: Withdraw a small portion of the granulation from the blender, add ingredient 9 through a No. 30 screen, mix well and return the mixture to the blender. Continue to tumble blend for an additional 5 minutes.

Step F: Compress tablets at 10-12 Kp hardness and about 0.225 inch guage using appropriate tooling.

Example 9

Sustained-release Tablet Formulation

To prepare 1000 sustained-release tablets each containing 450 mg of gemfibrozil and 40 mg of HMG-CoA reductase inhibitor, the method of Example 8 was employed utilizing the following ingredients.

Ingredients

1. Gemfibrozil, 450 g.
2. HMG-CoA reductase inhibitor, 40 g.
3. Microcrystalline cellulose type CL611, 35 g.
4. Cross carmellose sodium NF type A, 5 g.
5. Eudragit E30D, 180 g.
6. Purified water, 35 ml.
7. Talc USP, 5 g.
8. Cross carmellose Na NF type A, 7 g.
9. Ca stearate NF, 5 g.

Example 10

Sustained-release Tablet Formulation

To prepare 1000 sustained-release tablets each containing 600 mg of gemfibrozil and 20 mg of HMG-CoA reductase inhibitor, the method of Example 8 was employed utilizing the following ingredients.

Ingredients

1. Gemfibrozil, 600 g.
2. HMG-CoA reductase inhibitor, 20 g.
3. Microcrystalline cellulose type CL611, 46 g.
4. Cross carmellose sodium NF type A, 7 g.
5. Eudragit E30D, 240 g.
6. Purified water, 47 ml.
7. Talc USP, 7 g.
8. Cross carmellose sodium NF type A, 75 g.
9. Ca stearate NF, 7 g.

Example 11

Sustained-release Tablet Formulation

To prepare 1000 sustained-release tablets each containing 600 mg of gemfibrozil and 30 mg of HMG-CoA reductase inhibitor, the method of Example 8 was employed utilizing the following ingredients.

Ingredients

1. Gemfibrozil, 600 g.
2. HMG-CoA reductase inhibitor, 30 g.
3. Microcrystalline cellulose type CL611, 46 g.
4. Cross carmellose sodium NF type A, 7 g.
5. Eudragit E30D, 240 g.
6. Purified water, 47 ml.
7. Talc USP, 7 g.
8. Cross carmellose sodium NF type A, 75 g.
9. Ca stearate NF, 7 g.

Example 12

Sustained-release Tablet Formulation

To prepare 1000 sustained-release tablets each containing 900 mg of gemfibrozil and 20 mg of HMG-CoA reductase inhibitor, the method of Example 8 was employed utilizing the following ingredients.

Ingredients

1. Gemfibrozil, 900 g.
2. HMG-CoA reductase inhibitor, 20 g.
3. Microcrystalline cellulose type CL611, 75 g.
4. Cross carmellose sodium NF type A, 10 g.
5. Eudragit E30D, 240 g.
6. Purified water, 70 ml.
7. Talc USP, 10 g.
8. Cross carmellose sodium NF type A, 75 g.
9. Ca stearate NF, 10 g.

Pharmaceutical compositions for manufacturing of capsules or tablets for immediate release are prepared in general by blending the milled or microcrystalline active ingredients gemfibrozil and HMG-CoA reductase inhibitor with carriers, preferred silicon dioxide, starch, cross carmellose and/or hydroxyproylcellulose, granulated with water or aqueous polysorbate solution, dried at temperatures between 20 to 60°C. After drying the product is milled again and filled in capsules or is mixed with carriers and/or disintegrating agents, preferred with silicone dioxide, celluloses,talc, cross carmellose and/or stearates and pressed to tablets. To obtain retarded formulations retarding materials like polyacrylates may be added.

## Claims

1. A pharmaceutical composition comprising
a first component comprising
5-(2,5-dimethylphenoxy)-2,2-dimethylpentanoic acid or a lower alkyl ester or pharmaceutically acceptable base addition salt thereof together with
a second component comprising an HMG-CoA reductase inhibitor selected from

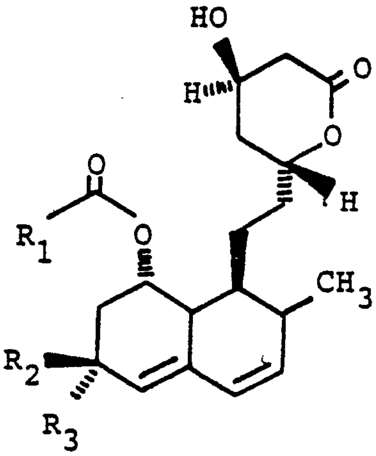

or

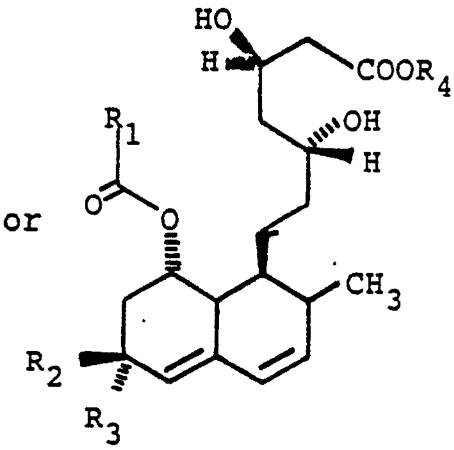

I II

where $R_1$ is selected from

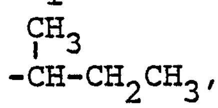, and

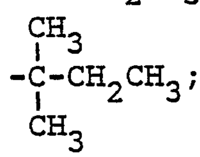;

$R_2$ is hydrogen or hydroxy;

$R_3$ is hydrogen or methyl; and

$R_4$ is lower alkyl or hydrogen or a pharmaceutically acceptable base addition salt thereof;

together with a pharmaceutically acceptable carrier; said first and second components being present in a weight ratio of respectively between 5:1 and 200:1.

2. A pharmaceutical composition as defined in Claim 1 wherein said second component is selected from

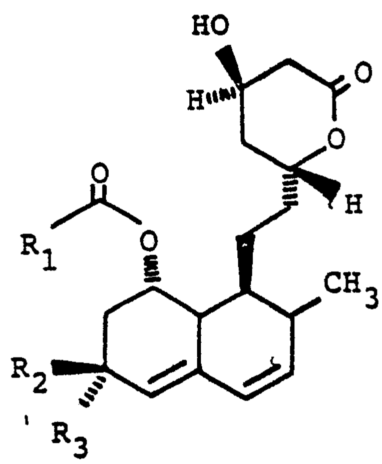

wherein $R_1$, $R_2$, and $R_3$ are as defined therein.

3. A pharmaceutical composition as defined in Claim 1 wherein said second component is selected from

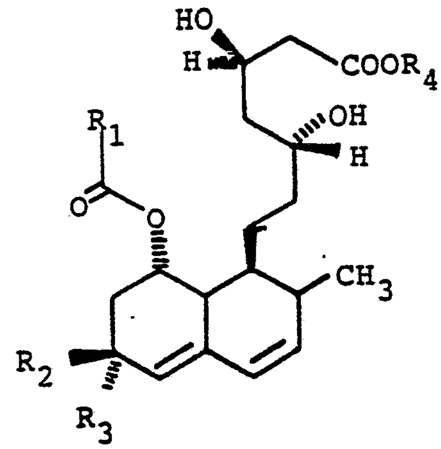

wherein $R_1$, $R_2$, $R_3$ and $R_4$ are as defined therein.

4. A pharmaceutical composition as defined in Claim 2 wherein said HMG-CoA reductase inhibitor is [1S-[1α(R*),3α,7β,8β(2S*,4S*),8aβ]]-1,2,3,7,8,8a-hexahydro-3,7-dimethyl-8-[2-(tetrahydro-4-hydroxy-6-oxo-2H-pyran-2-yl)ethyl]-1-naphthalenyl 2-methylbutanoate.

5. A pharmaceutical composition as defined in Claim 3 wherein said HMG-CoA reductase inhibitor is [1S-[1α(βS*,δS*),2α,6β,8β(R*)8aα]]-1,2,6,7,8,8a-hexahydro-β,δ-dihydroxy-2,6-dimethyl-8-(2-methyl-1-oxobutoxy)-1-naphthaleneheptanoic acid or a lower alkyl ester or pharmaceutically acceptable base addition salt thereof.

6. A pharmaceutical composition as defined in Claim 3 wherein said HMG-CoA reductase inhibitor is [1S-[1α,3α,7β,8β(2S*,4S*)8aβ]]-1,2,3,7,8,8a-hexahydro-3,7-dimethyl-8-[2-(tetrahydro-4-hydroxy-6-oxo-2H-pyran-2-yl)ethyl]-1-naphthalenyl 2,2-dimethylbutanoate.

7. A pharmaceutical composition as defined in Claim 3 wherein said HMG-CoA reductase inhibitor is [1S-[1α(βS*,δS*),2α,6β,8β,8aα]]-8-(2,2-dimethyl-1-oxobutoxy)-1,2,6,7,8,8a-hexahydro-β,δ-dihydroxy-2,6-dimethyl-1-naphthaleneheptanoic acid or a lower alkyl ester or pharmaceutically acceptable base addition salt thereof.

8. A pharmaceutical composition as defined in Claim 3 wherein said HMG-CoA reductase inhibitor is [1S-[1α(R*),3β,7β,8β(2S*,4S*),8aβ]]-1,2,3,7,8,8a-hexahydro-3-hydroxy-7-methyl-8-[2-(tetrahydro-4-hydroxy-6-oxo-2H-pyran-2-yl)ethyl]-1-naphthalenyl 2-methylbutanoate.

9. A pharmaceutical composition as defined in Claim 3 wherein said HMG-CoA reductase inhibitor is [1S-[1α(βS*,δS*),2α,6α,8β(R*),8aα]]-1,2,6,7,8,8a-hexahydro-β,δ,6-trihydroxy-2-methyl-8-(2-methyl-1-oxobutoxy)-1-naphthaleneheptanoic acid or a lower alkyl ester or pharmaceutically acceptable base addition salt thereof.

10. A pharmaceutical composition as defined in Claim 3 wherein said HMG-CoA reductase inhibitor is [1S-[1α(R*),7β,8β(2S*,4S*),8aβ]]-1,2,3,7,8,8a-hexahydro-7-methyl-8-[2-(tetrahydro-4-hydroxy-6-oxo-2H-pyran-2-yl)ethyl]-1-naphthanlenyl 2-methylbutanoate.

11. A pharmaceutical composition as defined in Claim 3 wherein said HMG-CoA reductase inhibitor is [1S-[1α(βS*,δS*),2α,8β(R*),8aα]]-1,2,6,7,8,8a-hexahydro-β,δ-dihydroxy-2-methyl-8-(2-methyl-1-oxobutoxy)-1-naphthaleneheptanoic acid or a lower alkyl ester or pharmaceutically acceptable base addition salt thereof.

12. A pharmaceutical composition in accordance with Claim 1 in capsule form containing between 300 and 900 milligrams of 5-(2,5-dimethylphenoxy)-2,2-dimethylpentanoic acid or a lower alkyl ester or pharmaceutically acceptable base addition salt thereof together with from 5 to 80 milligrams of said HMG-CoA reductase inhibitor.

13. A pharmaceutical composition in accordance with Claim 1 in immediate release tablet form containing between 300 and 900 milligrams of 5-(2,5-dimethylphenoxy)-2,2-dimethylpentanoic acid or a lower alkyl ester or pharmaceutically acceptable base addition salt thereof together with 5 to 80 milligrams of said HMG-CoA reductase inhibitor.

14. A pharmaceutical composition in accordance with Claim 1 in sustained release tablet form containing between 300 and 900 milligrams of 5-(2,5-dimethylphenoxy)-2,2-dimethylpentanoic acid or a lower alkyl ester or pharmaceutically acceptable base addition salt thereof together with 5 to 80 milligrams of said HMG-CoA reductase inhibitor.

15. Process for the manufacturing of pharmaceutical compositions according to claims 1 to 14 comprising blending the milled or microcrystalline active ingredients gemfibrozil and HMG-CoA reductase inhibitor with milled or microcristalline carriers, and if desired with retarding agents, granulating the mixed compounds, drying at temperatures between 20 to 60°C, milling the dried product again and filling into capsules or mixing the dried and milled product with carriers and/or disintegrating agents to obtain tablets.